Europäisches Patentamt

European Patent Office

Office européen des brevets

(11) Publication number: **0 440 193 A2**

# EUROPEAN PATENT APPLICATION

(21) Application number: 91101205.2

(22) Date of filing: 30.01.91

(51) Int. Cl.⁵: **G01N 33/546**, G01N 33/576, G01N 33/78, G01N 33/569, G01N 33/577

(30) Priority: 31.01.90 JP 19000/90

(43) Date of publication of application:
07.08.91 Bulletin 91/32

(84) Designated Contracting States:
**CH DE ES FR GB IT LI NL**

(71) Applicant: **FUJIREBIO INC.**
**6-7, Shimoochiai 4-chome Shinjuku-ku**
**Tokyo 161(JP)**

(72) Inventor: **Kasahara, Yasushi**
**24-6, Hijirigaoka 4-chome**
**Tama-shi, Tokyo(JP)**
Inventor: **Ashihara, Yoshihiro**
**2-402, Fuchudanchi, 28-1, Harumicho**
**1-chome**
**Fuchu-shi, Tokyo(JP)**

(74) Representative: **Hansen, Bernd, Dr.**
**Dipl.-Chem. et al**
**Hoffmann, Eitle & Partner Patent- und**
**Rechtsanwälte Arabellastrasse 4**
**W-8000 München 81(DE)**

(54) Immunochemical assay method with plural items.

(57) There is disclosed an immunochemical assay method capable of simultaneously measuring plural items which comprises contacting mixed particles each having an average diameter of 0.1 to 1.0 µm and containing at least two kinds of particles, to which different kind of an antigen or antibody to be assayed is separately bound, with a specimen in a single reaction phase.

EP 0 440 193 A2

## IMMUNOCHEMICAL ASSAY METHOD WITH PLURAL ITEMS

### BACKGROUND OF THE INVENTION

This invention relates to an immunochemical assay method of a virus antibody or a hormone with multichannel simultaneously.

As a prior method, it has been generally employed a method in which each item to be detected is separately measured to obtain the result. On the other hand, in an immunological assay method, as a method for examining presence or absence of multichannel antibodies or antigens simultaneously, there have been known the method in which antigens or antibodies to be bound to a solid phase are previously mixed and then bound to the solid phase to use it for measuring, or the immunoassay method in which a solid phase of polystyrene balls having a diameter of 1 to 10 mm, to which plural kinds of antibodies or antigens are separately bound, is used.

However, the method of assaying respective items separately in accordance with each item to be detected requires much amounts of specimen and is not suitable for a patient to whom an amount of the specimen is limited such as a newly-born infant. Also, much costs are required for effecting the method since costs in accordance with the number of items are required.

Also, in the above simultaneous multichannel method, a bound capacity of the solid phase is small and thus it is difficult to determine multichannel components simultaneously. Further, when the reaction time is elongated, it is also difficult to match degrees of detecting signals at substantially the same level between two or more items to be detected since concentrations of the items to be measured in the specimen are different from each other. On the other hand, in the immunoassay method using a solid phase wherein antibodies or antigens are separately bound to polystyrene balls having 1 to 10 mm, a volume of the solid phase itself becomes large so that greater amount of specimens are required.

### SUMMARY OF THE INVENTION

In order to solve these problems, the present inventors have intensively studied, and as the results, they have found that by using a solid phase prepared by binding respective antibodies to be measured, or an antibody to an antigen to particles having a diameter of 0.1 to 1.0 $\mu$m and mixing them so as to become the same signal/noise (S/N), and by employing a detecting system having high sensitivity, simultaneous multichannel immunochemical assay method can be developed to accomplish the present invention.

That is, the present invention is an immunochemical assay method capable of measuring plural items simultaneously which comprises preparing at least two kinds of particles each of which has an average diameter of 0.1 to 1.0 $\mu$m and to which one kind of an antigen or antibody capable of reacting with an antibody or antigen to be assayed is bound, mixing said at least two kinds of particles to which different kinds of the antigens or antibodies are separately bound, and reacting the particles mixture with a specimen to be assayed

### DESCRIPTION OF THE PREFERRED EMBODIMENT

A subject to be measured in the present invention is a virus antibody or a hormone contained in a specimen. The kind of the specimen is not limited and the specimen may include, for example, plasma, serum, erythrocyte extracted solution, urea and whole blood extracted solution. Items to be assayed in the present invention include infectious diseases such as hepatitis A, hepatitis B, hepatitis C, HIV, ATLA and TPHA and hormones such as T3, T4, Free T4 and 17OHP.

In the present invention, as the antigen to be bound to particles, there may be mentioned hepatitis A virus, hepatitis B virus, hepatitis C virus, T3 antigen, T4 antigen, free T4 antigen, human immunodefficiency virus, human adult T cell lymph curpuscle virus and treponeme pallidum, and at least two of them can be used in combination.

As the antibody to be bound to particles, there may be mentioned anti-thyroid tropic hormone antibody, anti-17-hydroxyprogesteron antibody, anti T3 antibody, anti T4 antibody, anti free T4 antibody, anti-hepatitis A virus antibody (HAAb), anti-hepatitis B virus antibody (HBAb) and anti-hepatitis C virus antibody (HCAb), and at least two of them can be used in combination.

Preparation of antibody

A polyclonal antibody to be used in the present invention can be obtained by injecting an antigen with an adjuvant to warm-blooded animal such as rabbit, goat, horse, guinea pig and chicken with an amount of 0.3 to 2 mg per 1 kg of body weight once to several times to ones back hypodermically, foot pat or thigh muscle and then treating its serum. Also, a monoclonal antibody can be produced by injecting an antigen with a Freund's adjuvant to a mouse with an amount of 0.01 to 0.5 mg per mouse, delivering spleen at a state of high antibody value, hybridizing the spleen cell with mouse myeloma cells in polyethylene glycol, selecting cells producing the said antibody from these cells, increasing the cells as monoclone cells, and cell culturing in a large scale in a mouse abdomen or culturing broth (see "Monoclonal Antibody and Cancer", published by Science Forum K.K., 1985).

As an immunochemical assay method in the present invention, either of the radioimmunoassay method or the enzyme immunoassay method is employed, simultaneous multichannel assay can be effectively carried out qualitatively or quantitatively. In the enzyme immunoassay method according to the present invention, for example, for detection of a virus, various virus antigens are bound to the above particle. And, in order to determine a mixing ratio of particles, an antigen is previously measured by each subject to be detected. Next, particles are diluted and mixed so as to have the same S/N. To the particles is added a predetermined amount of a specimen and the reaction is carried out for one minute to 30 hours and then washing the particles. To the particles is added a predetermined amount of an enzyme-labelled anti-human IgG antibody, the reaction is carried out for one minute to 30 hours and then washing the particles. The reaction temperatures of the above reactions are each 4 °C to 40 °C, preferably 25 °C to 38 °C. After washing, an amount of the antibody in the specimen can be qualitatively or quantitatively detected by measuring activity of an amount of an antibody-bound enzyme bound to particles after addition of an enzyme substrate. The enzyme to be used in the assay method of the present invention is a peroxidase, an alkaliphosphatase, $\beta$-galactosidase and glucoseoxidase. At this time, it is needless to say that the substrate should be one suitable for an enzyme to be used. For example, there may be mentioned ABTS (2,2'-azino-bis(3-ethylbenzothiazoline-6-sulfonic acid)), luminol-$H_2O_2$ (for a peroxidase), p-nitrophenylphosphate, methylumbelliferyl phosphate and AMPPD (3-(2'-pyro-tricyclo[3.3.1.1$^{3,7}$]decan)-4-methoxy-4-(3''-phosphoryloxy)phenyl-1,2-dioxetane disodium salt) (for alkaliphosphatase), p-nitrophenyl-$\beta$-o-glactose, methylumbelliferyl-$\beta$-o-galactose and AMGPD (3-(2'-pyro-tricyclo[3.3.1.1$^{3,7}$]decan)-4-methoxy-4-(3-(2'-$\beta$-O-galactopyranosil)-phenyl-1,2-dioxetane) (for $\beta$-galactosidase). After enzymatic reaction, the measurement can be carried out by reacting at 4 °C to 40 °C under heating for 1 minute to 18 hours, and then measuring an amount of color, fluorescence or luminescence generated.

Also, the radioimmunoassay method in the immunochemical assay method of the present invention is carried out by labelling a radioisotope such as $^{125}$I in place of the above enzyme label. Operations when effecting the above method are quitely the same with the aforesaid enzyme immunoassay method except for measuring radioactivity.

When binding an antibody or an antigen to particles, the physical absorption method or the chemical absorption method can be employed. For example, when carboxymethylated particles are used, an agent can be prepared by adding a water-soluble carbodiimide to 0.1 to 1 mg of an antibody solution (pH 5 to 7) and reacting them for 1 to 5 hours. For effecting an immuno reaction, the reaction can be carried out by adding a sample to the aforesaid antibody-immobilized particles and then reacting them at 4 °C to 40 °C, preferably 20 °C to 38 °C for 1 to 18 hours. Thereafter, washing is carried out by adding a physiological salt solution or distilled water, adding radiolabelled antibody to the particles, followed by reacting at 4 °C to 40 °C, preferably 20 °C to 38 °C for 1 to 18 hours and washing with a physiological salt solution or distilled water, and then countering its radioactivity. A scintillation counter can be used for the measurement.

Also, the assay method of the present invention may be carried out by the chemiluminescent assay method in which isoluminol or acridine ester is labelled, or the fluorescent immunoassay method in which fluoresceine or rhodamine is labelled. During the procedures, labelling of a labelling substance can be easily carried out by employing the active ester method or the isocyanate method (see "Enzyme immunoassay method" (published by Igaku Shoin, 1987)).

According to the present invention, different kind of some antibodies or antigens contained in human serum or urine can be measured simultaneously.

EXAMPLES

In the following, the present invention will be explained by referring to Examples in more detail.

Reference example 1

Preparation of reagent for measuring peroxidase activity

(i) To tolicin-NaOH buffer (pH = 8.4, 50 mM) were added 1.0 mM of phenothiazine-10-yl-propylsulfonate and 0.1 mM of phenolindophenol.

(ii) A luminol-DMSO solution was added to a 1 mM of hydrogen peroxide aqueous solution so as to become the initial concentration of 750 $\mu$M.

Example 1

1-i Preparation of anti-TSH mouse IgG-bound magnetic particles

To 4 ml of a 5 % ferrite coated particles (0.3 $\mu$m)-dispersed aqueous dispersion (20 mM phosphate buffer, pH 3.5) was added 1 ml of anti-TSH mouse IgG (5 mg/ml) and the mixture was stirred by an end-over-end mixer at room temperature overnight. This particles-dispersed liquor was separated by a magnet with a surface magnetic field of 3000 gausses to supernatant and particles and then the supernatant was removed. The particles were washed five times with a 2 % BSA solution (0.1 M Tris-hydrochloric acid, 1 mM magnesium chloride and 0.1 mM zinc chloride, pH 7.5) and then dispersed in 5 ml of the similar BSA solution to prepare magnetic particles.

1-ii Preparation of anti-17-hydroxyprogesterone rabbit IgG-bound magnetic particles (Anti-17-OHP IgG bound)

To 4 ml of a 5 % ferrite coated particles (0.3 $\mu$m)-dispersed aqueous dispersion (20 mM phosphate buffer, pH 3.5) was added 1 ml of anti-17-hydroxyprogesterone rabbit IgG (5 mg/ml) and the mixture was stirred by an end-over-end mixer at room temperature overnight. This particles-dispersed liquor was separated by a magnet with a surface magnetic field of 3000 gausses to supernatant and particles and then the supernatant was removed. The particles were washed five times with a 2 % BSA solution (0.1 M Tris-hydrochloric acid, 1 mM magnesium chloride and 0.1 mM zinc chloride, pH 7.5) and then dispersed in 5 ml of the similar BSA solution to prepare magnetic particles.

These two kinds of magnetic particles were mixed with 0.02 % of anti-TSH mouse IgG particles and 0.008 % of anti-17-OHP rabbit IgG particles to prepare particles for detecting TSH and 17-OHP.

To 200 $\mu$l of ferrite particles dispersion prepared in Example 1 for detecting hormones were added 20 $\mu$l of a specimen and 20 $\mu$l of a mixture of peroxidase-bound TSH and peroxidase-bound 17-OHP (0.5 $\mu$g/ml) and the mixture was allowed to stand at room temperature for 30 minutes.

The tube containing the above mixture was contacted with a magnet having a surface magnetic field of 3000 gausses to collect ferrite particles magnetically and the supernatant was removed by decantation. Thereafter, 1 ml of a 0.04 % physiological salt solution was added and the mixture was stirred. This tube was also contacted with the same magnet as mentioned above to remove the supernatant by decantation. This operations were repeated three times. To this tube containing the particles was added 200 $\mu$l of an agent for detecting a peroxidase activity prepared in Reference example 1. Subsequently, 135 $\mu$l of the solution (i) prepared in Reference example 1 was added thereto and reacted at 37 °C for 30 minutes. Thereafter, the tube was placed in a photocounter to measure the activity of the peroxidase. During 10 seconds of measurement, luminescence for 4 seconds were integrated.

The results measured by using each specimen and standard antigen are shown in Table 1.

4

## Table 1

### Count (5 seconds)

| | | |
|---|---|---|
| TSH 0 μU/ml | 380000 | |
| TSH 5 μU/ml | 100000 | |
| TSH 20 μU/ml | 50000 | |
| TSH 40 μU/ml | 10000 | |
| 17-OHP 0 μU/ml | 382000 | |
| 17-OHP 3 μU/ml | 115000 | |
| 17-OHP 10 μU/ml | 85000 | |
| 17-OHP 40 μU/ml | 31000 | |
| (Specimen) | | (Judgement) |
| A | 365000 | Normal |
| B | 380000 | Normal |
| C | 220000 | Reexamination |
| D | 100000 | Definitive examination |
| E | 150000 | Definitive examination |
| F | 375000 | Normal |

Example 2

2-i Preparation of HIV, ATLA or HBV-bound ferrite particles

To 4 ml of a 5 % ferrite coated particles (0.3 μm)-dispersed aqueous dispersion (20 mM phosphate buffer, pH 3.5) was added 1 ml of each antigen (5 mg/ml) and the mixture was stirred by an end-over-end mixer at room temperature overnight. This particles-dispersed liquor was separated by a magnet with a surface magnetic field of 3000 gausses to supernatant and particles and then the supernatant was removed. The particles were washed five times with a 2 % BSA solution (0.1 M Tris-hydrochloric acid, 1 mM magnesium chloride and 0.1 mM zinc chloride, pH 7.5) and then dispersed in 5 ml of the similar BSA solution to prepare magnetic particles.

The above particles sensitized with each antigen of HIV, ATLA or HBV were diluted with a 2 % BSA solution so as to become a final concentration of 0.01 % in the case of HIV, 0.008 % in ATLA and 0.002 % in HBV to prepare ferrite particles for screening.

20 μl of human serum was sampled and diluted with a 2 % BSA solution (0.1 M Tris-hydrochloric acid, 1 mM MgCl$_2$ and 0.1 mM ZnCl$_2$, pH 7.5) containing a 5 % rabbit serum, and 20 μl of this sample was placed in a tube. To this sample was added 350 μl of ferrite particles for screening prepared in Example 2, 2-i and reacted at room temperature for 30 minutes.

Ferrite particles were collected magnetically by contacting with a magnet having a surface magnetic field of 3000 gausses and the supernatant was removed by decantation. Thereafter, 1 ml of a 0.04 % physiological salt solution was added and the mixture was stirred. This tube was also contacted with the same magnet as mentioned above to remove the supernatant by decantation. This operations were repeated three times. With this tube was mixed 200 μl of an anti-human Ig-mouse IgG alkali phosphatase conjugate (conjugate concentration: 0.5 μg/ml, 0.1 M Tris-hydrochloric acid, 2 % BSA, 1 mM MgCl$_2$ and 0.1 mM ZnCl$_2$, pH 7.5), and reacted at room temperature for 20 minutes.

This tube was, contacted with a magnet having a surface magnetic field of 3000 gausses to collect ferrite particles magnetically and the supernatant was removed by decantation. Thereafter, 1 ml of a 0.04 % physiological salt solution was added and the mixture was stirred. This tube was also contacted with the

EP 0 440 193 A2

same magnet as mentioned above to remove the supernatant by decantation. This operations were repeated three times. To this tube containing the particles was added 200 $\mu$l of a substrate solution (0.1 mM $ZnCl_2$, pH 9.8) containing 100 $\mu$g/ml of 3-(2'-pyro-tricyclo[3.3.1.1$^{3,7}$]decan)-4-methoxy-4-(3''-phosphoryloxy)-phenyl-1,2-dioxetane disodium salt (AMPPD) and reacted at room temperature. After effecting the reaction for 5 minutes, the sample was measured by a luminometer (manufactured by Belthold Co.). The results are shown in Table 2. In Table 2, a specimen having a cut off value becomes 5 (=(signal of the specimen)/-(signal of negative control)) is judged as positive.

## Table 2

| | Count (x10⁴/5 seconds) | (Judgement) |
|---|---|---|
| Rabbit serum | 0.5 | |
| Human normal serum A | 0.8 | |
| Human normal serum B | 0.9 | |
| Human normal serum C | 1.1 | |
| Specimen E | 0.8 | − |
| Specimen F | 25.0 | + |
| Specimen G | 5.2 | + |
| Specimen H | 1.8 | ± |
| Specimen I | 9.5 | + |
| Specimen J | 10.6 | + |

Example 3

3-i Preparation of HIV-1 or HTLV-I-bound ferrite particles

In 3.43 ml of a 8 M urea solution (50 mM Tris-hydrochloric acid buffer solution, pH 7.5) was suspended 6.5 mg of ferrite coated particles washed with distilled water, and then, to the suspension was added 2.34 ml of HIV-1 antigen (36.6 $\mu$g/ml) and the mixture was stirred at room temperature overnight by an end-over-end mixer. Similarly, in 12.5 ml of a 50 mM acetic acid buffer solution (pH 5.5) was suspended 23.7 mg of ferrite coated particles, and then 2.5 ml of HTLV-I antigen (400 $\mu$g/ml) was added thereto and the mixture was stirred at room temperature overnight by an end-over-end mixer. These particle-dispersions were each separated by contacting with a magnet having a surface magnetic field of 3000 gausses to a supernatant and particles and then the supernatant was removed. The particles were washed four times with a 2 % BSA solution (0.1 M Trishydrochloric acid and 1 mM magnesium chloride, pH 7.5) and finally washed with a 2 % BSA solution containing a 2 % rabbit serum. These particles were mixed and dispersed in 700 ml of a 2 % BSA solution containing the similar 2 % rabbit serum, stirred two nights at 37 °C by an end-over-end mixer to prepare ferrite particles for screening.

20 $\mu$l of human serum was sampled and diluted with a 2 % BSA solution (0.1 M Tris-hydrochloric acid and 1 mM $MgCl_2$, pH 7.5) containing a 50 % rabbit serum, and 20 $\mu$l of this sample was placed in a tube. To this sample was added 350 $\mu$l of ferrite particles for screening prepared in Example 3, 3-i and reacted at 37 °C for 30 minutes.

Ferrite particles were collected magnetically by contacting with a magnet having a surface magnetic field of 3000 gausses and the supernatant was removed by decantation. Thereafter, 1 ml of a 0.04 % physiological salt solution was added and the mixture was stirred. This tube was also contacted with the same magnet as mentioned above to remove the supernatant by decantation. This operations were repeated three times. With this tube was mixed 250 $\mu$l of an anti-human Ig-mouse IgG alkali phosphatase conjugate (conjugate concentration: 0.2 $\mu$g/ml, 0.1 M Tris-hydrochloric acid, 2 % BSA, 2 % rabbit serum, 1

mM $MgCl_2$ and 0.1 mM $ZnCl_2$, pH 7.5), and reacted at 37 °C for 10 minutes.

Next, this tube was contacted with a magnet having a surface magnetic field of 3000 gausses to collect ferrite particles magnetically and the supernatant was removed by decantation. Thereafter, 1 ml of a 0.04 % physiological salt solution was added and the mixture was stirred. This tube was also contacted with the same magnet as mentioned above to remove the supernatant by decantation. This operations were repeated three times. To this tube containing the particles was added 300 $\mu l$ of a substrate solution (0.1 mM $ZnCl_2$, pH 10.0) containing 100 $\mu g/ml$ of 3-(2'-pyro-tricyclo[3.3.1.1$^{3,7}$]decan)-4-methoxy-4-(3''-phosphoryloxy)phenyl-1,2-dioxetane disodium salt (AMPPD) and reacted at 37 °C. After effecting the reaction for 5 minutes, the sample was measured by a luminometer (manufactured by ALOKA Co.).

Judgement of positive or negative was carried out based on the signal of the previously determined negative control serum as a standard. That is, by calculating the value (positive/negative value = P/N value) obtained by dividing the signal value obtained by a human serum which is a specimen into the signal value of a negative control serum, and the value is 5 or more, it had been judged as positive.

With respect to 27 samples of HIV-1 positive specimens and 25 samples of HTLV-I positive specimens which are confirmed to be positive by the Western•Blott method, indirect fluorescence antibody method or ELISA method, measurement was carried out for all the above samples by the above method. As the results, all the samples became P/N value of 5 or more to be judged as positive. Further, with respect to 558 examples of normal men which are confirmed to be negative by the similar method and rheumatoid patients, measurement was carried out by the above method. As the results, only 4 examples became P/N value of 5 or more but the remaining 554 examples became P/N value of less than 5 to be judged as negative.

Sensitivity and specificity were calculated by the above results to give the sensitivity = 100 % and the specificity = 99.3 %.

## Claims

1. An immunochemical assay method capable of measuring plural items simultaneously which comprises preparing at least two kinds of particles each of which has an average diameter of 0.1 to 1.0 $\mu m$ and to which one kind of an antigen or antibody capable of reacting with an antibody or antigen to be assayed is bound, mixing said at least two kinds of particles to which different kinds of the antigens or antibodies are separately bound, and reacting the particles mixture with a specimen to be assayed.

2. The method according to Claim 1, wherein said assay method is carried out under a constant signal/noise (S/N) value by controlling an amount of antigen, an amount of antibody or an amount of particles to which an antigen or antibody is bound.

3. The method according to Claim 1, wherein said antigen to be bound to particles is at least two selected from hepatitis A virus, hepatitis B virus, hepatitis C virus, T3 antigen, T4 antigen, free T4 antigen, human immunodefficiency virus, human adult T cell lymph curpuscle virus and treponeme pallidum.

4. The method according to Claim 1, wherein said antibody to be bound to particles is at least two selected from anti-thyroid tropic hormone antibody, anti-17-hydroxyprogesteron antibody, anti T3 antibody, anti T4 antibody, anti free T4 antibody, anti-hepatitis A virus antibody (HAAb), anti-hepatitis B virus antibody (HBAb) and anti-hepatitis C virus antibody (HCAb).

5. The method according to Claim 1, wherein said particle is a magnetic particle or a latex.

6. The method according to Claim 4, wherein said antibody is a mouse monoclonal antibody.

7. The method according to Claim 1, wherein said immunochemical assay method is an enzyme immunoassay method.

8. The method according to Claim 1, wherein said immunochemical assay method is a radio immunoassay method.

9. The immunoassay method according to Claim 7, wherein said enzyme immunoassay method is carried out by binding virus antigen to particles to prepare at least two kinds of particles, diluting each of particles to have the same S/N, mixing at least two kinds of particles, adding a specimen to the mixture of particles to react them, washing the particles, adding a predetermined amount of an enzyme-labelled anti-human IgG antibody to the reaction mixture to further react them, separating unreacted enzyme-labelled antibody, washing the particles, and adding an enzyme substrate to the particles to measure an activity of an antibody-bound enzyme.

10. The immunoassay method according to Claim 9, wherein said enzyme substrate is selected from the group consisting of ABTS (2,2'-azino-bis(3-ethylbenzothiazoline-6-sulfonic acid)) and luminol-$H_2O_2$ for peroxidase; p-nitrophenylphosphate, methylumbelliferyl phosphate and (2'-pyro-tricyclo[3.3.1.1$^{3,7}$]-decan)-4-methoxy-4-(3''-phosphoryloxy)phenyl-1,2-dioxetane disodium salt for alkaliphosphatase; and p-nitrophenyl-$\beta$-O-glactose, methylumbelliferyl-$\beta$-O-galactose and AMGPD (3-(2'-pyro-tricyclo-[3.3.1.1$^{3,7}$]decan)-4-methoxy-4-(3-(2'-$\beta$-O-galactopyranosil)-phenyl-1,2-dioxetane) for $\beta$-galactosidase.

11. The immunoassay method according to Claim 10, wherein said enzyme substrate is 2'-pyro-tricyclo-[3.3.1.1$^{3,7}$]decan)-4-methoxy-4-(3''-phosphoryloxy)phenyl-1,2-dioxetan disodium salt.